# EUROPEAN PATENT APPLICATION

(11) **EP 0 768 303 A1**
(43) Date of publication of application: **16.04.1997**
(21) Application number: 95922744.8
(22) Date of filing: 23.06.1995
(51) Int. Cl.: C07D 215/56, C07D 513/00

(54) **OPTICALLY ACTIVE QUINOLINECARBOXYLIC ACID DERIVATIVE AND PROCESS FOR PRODUCING THE SAME**

(30) Priority: 27.06.1994 JP 144892/94
(71) Applicant: NIPPON SHINYAKU COMPANY, LIMITED, Minami-ku Kyoto-shi Kyoto 601 (JP)
(72) Inventor: SEGAWA, Jun, Nara 631 (JP); MATSUOKA, Masato, Kyoto 606 (JP); AMIMOTO, Isao Nagaoka-ryo Nippon Shinyaku Co., Ltd, Nagaokakyo-shi Kyoto 617 (JP)
(74) Representative: Strych, Werner Maximilian Josef, Dr.
(86) International application number: JP9501255
(87) International publication number: WO9600217

(57) **Abstract**

This invention provides a process for producing compound [Ia] and [Ib] which comprises reacting compound [III] with a chiral carboxylic acid or amino acid, or a reactive derivative thereof, reacting the resulting compound [II] with a chlorinating agent, and separating diastereomers from the resulting diastereomer mixture. 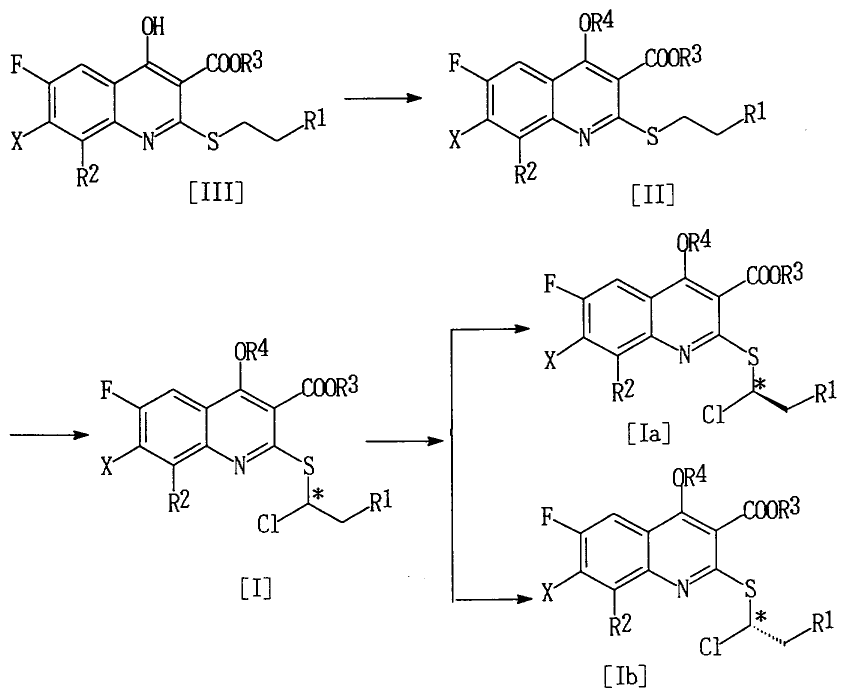 wherein R¹ typically represents hydrogen or halogen; R² typically represents hydrogen; R³ typically represents alkyl; R⁴ represents chiral acyl or amino residue; X represents halogen, substituted or unsubstituted cyclic amino, or phenyl.

## Description

### TECHNICAL FIELD

The present invention relates to an intermediate compound useful for the production of optically active thiazetoquinolinecarboxylic acid derivatives (e.g. Japanese Kokai Tokkyo Koho H3-218383, among others) which are of value as antimicrobial agents and to a process for producing the same.

### BACKGROUND ART

For the purpose of obtaining an optically active 6-fluoro-7-substituted-4-oxo-4H-[1,3]thiazeto[3,2-a]quinoline-3-carboxylic acid alkyl ester derivative [IV] 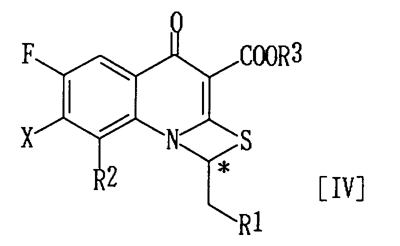 (wherein R¹, R², R³, and X are as defined hereinafter; * stands for an asymmetric carbon) which is an important intermediate for the production of optically active thiazetoquinolinecarboxylic acid derivatives of value as antimicrobial agents, the inventors of the present invention created an optically active 2-(1-chloro-2-acyloxyethyl)thio-6-fluoro-7-substituted-4-acyloxyquinoline-3-carboxylic acid ethyl ester derivative and already filed a patent application (WO 94/14819). This compound is a compound having a single asymmetric carbon atom and is separated by an enzymatic process but the enzymatic resolution of optically active compounds has drawbacks such as difficulty in mass processing, low yield, and a protracted process.

### DISCLOSURE OF INVENTION

Paying attention to the usefulness of the optically active 6-fluoro-7-substituted-4-oxo-4H-[1,3]thiazeto[3,2-a]quinoline-3-carboxyl acid alkyl ester derivative [IV] and to obviate the disadvantages of the known process for producing an optically active 2-(1-chloro(-2-substituted)ethyl)thio-6-fluoro-7-substituted-4-acyloxyquinoline-3-carboxylic acid alkyl ester derivative, in which the 2-position of the ethyl moiety of its ethylthio group may be substituted, which is an intermediate of [IV], the inventors of the present invention endeavored to develop a novel and advantageous production process.

The object of the present invention is to provide an optically active 2-(1-chloro(-2-substituted)ethyl)thio-6-fluoro-7-substituted-4-acyloxyquinoline-3- carboxylic acid alkyl ester derivative and a process for producing the same.

In the course of exploring a variety of production processes, the inventors found that an optically active 2-(1-chloro(-2-substituted)ethyl)thio-6-fluoro-7-substituted-4-acyloxyquinoline-3-carboxylic acid alkyl ester derivative having a chiral substituent at 4-position can be produced easily with high efficiency on a high production scale by introducing a chiral substituent group into the 4-position of a 2-(-2-substituted)ethyl)thio-6-fluoro-7-substituted-4-hydroxyquinoline-3-carboxylic acid alkyl ester derivative, chlorinating the ethyl group at 2-position, and carrying out separation by a suitable technique.

The present invention is directed to optically active quinolinecarboxylic acid derivatives of the following general formula [Ia] and [Ib] 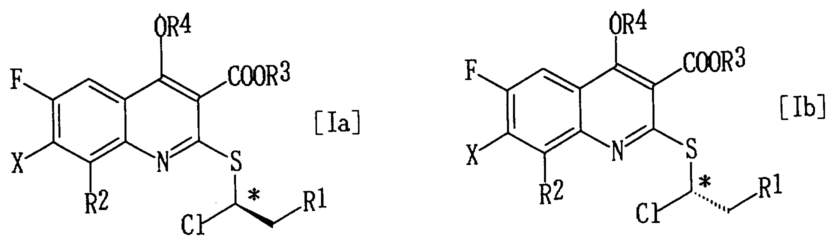 [wherein R¹ represents hydrogen, halogen, hydroxy, alkoxy, aryloxy, or acyloxy; R² represents hydrogen, alkyl, alkoxy, amino, or halogen; R³ represents alkyl; R⁴ represents chiral acyl; X represents halogen, substituted or unsubstituted cyclic amino, or phenyl].

The present invention is further directed to a process for producing an optically active quinolinecarboxylic acid derivative of general formula [Ia] or [Ib] 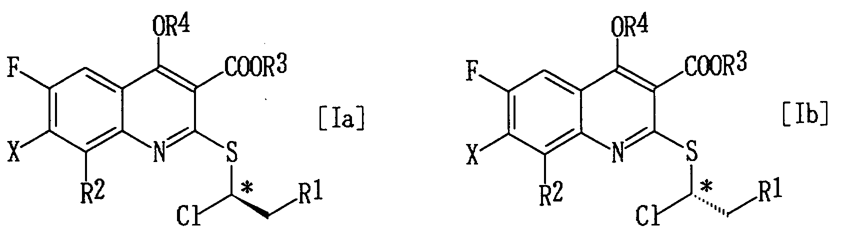 [wherein R¹, R², R³, R⁴, and X are as defined hereinbefore] which comprises reacting a compound of general formula [III] 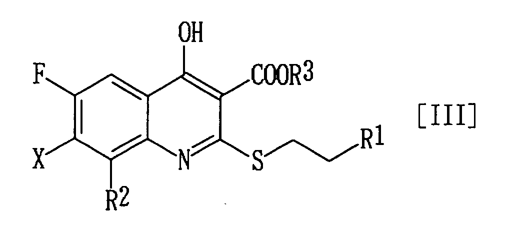 [wherein R¹, R², R³, and X are as defined above] with a chiral carboxylic acid or amino acid, or a reactive derivative thereof, treating the resulting compound of general formula [II] with a chlorinating agent 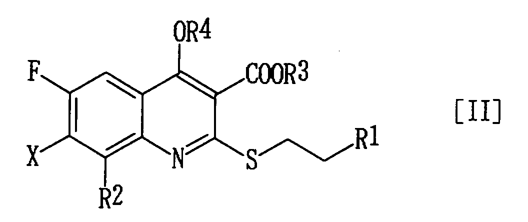 [wherein R¹, R², R³, R⁴, and X are as defined above], and separating each diastereomer from the resulting diastereomer mixture [I] 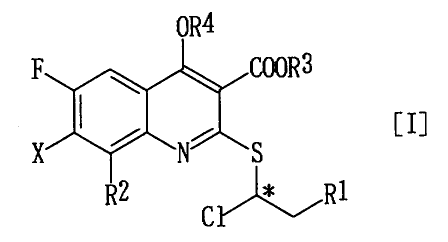 [wherein R¹, R², R³, R⁴, and X are as defined above].

The present invention is characterized in that a compound of general formula [III] is reacted with a chiral carboxylic acid or amino acid or a reactive derivative thereof to introduce a chiral substituent group, the reaction product is then chlorinated to generate a second asymmetric carbon atom, and the resulting diastereomers are separated.

The present invention is now described in detail.

Referring to general formulas [I] to [IV], the halogen represented by R¹, R², and X may for example be fluorine, chlorine, or bromine.

The alkoxy represented by R¹ and R² is preferably a straight-chain or branched-chain lower alkoxy group of 1-4 carbon atoms, thus including methoxy, ethoxy, n-propoxy, isopropoxy, n-butoxy, isobutoxy, sec-butoxy, and tert-butoxy.

The aryl of the aryloxy represented by R¹ is preferably an aryl group of 6-10 carbon atoms and may for example be unsubstituted or halogen-or alkoxy-substituted phenyl, α-naphthyl, or β-naphthyl. The halogen and alkoxy may be those mentioned above.

The acyl of the acyloxy represented by R¹ is preferably an acyl group of 1-4 carbon atoms and may for example be formyl, acetyl, propionyl, or butyryl.

The alkyl represented by R² and R³ is preferably a straight-chain or branched-chain alkyl group of 1-4 carbon atoms and may for example be methyl, ethyl, n-propyl, isopropyl, n-butyl, isobutyl, sec-butyl, or tert-butyl.

The chiral acyl represented by R⁴ can be any acyl group having an asymmetric carbon atom and, for example, branched alkylcarbonyl, branched aralkylcarbonyl, and amino acid residues can be employed. In case an amino acid residue is used, a residue whose amino group has been protected is used so that the reaction will not be interfered with. The chiral acyl includes but is not limited to 2-phenylpropionyl, 2-methoxy-2-phenylacetyl, O-benzyllactoyl, N-benzyloxycarbonyl-D-phenylglycyl, N-benzyloxycarbonyl-L-alanyl, N-benzoyl-L-prolyl, L-benzoyl-L-alanyl, N-methoxycarbonyl-D-glycyl, 2-acetoxy-2-phenylacetyl, and 2-methyloctanoyl. Above all else, acyl groups having an asymmetric carbon atom at 2-position, such as 2-phenylpropionyl and 2-methoxy-2-phenylacetyl, are preferred.

The cyclic amino group represented by X is a group having a 4- through 8-membered ring structure, which may optionally contain nitrogen, oxygen, or sulfur as a ring member. Such cyclic amino group includes but is not limited to 1-azetidinyl, 1-pyrrolidinyl, piperidino, 1-azepinyl, 1-azocinyl, 1-piperazinyl, 1-homopiperadinyl, 1-pyrrolinyl, morpholino, thiomorpholino, 3,7-diazabicyclo[3.3.0]oct-1(5)-en-3-yl, 4,7-diazaspiro[2.5]octen-7-yl, and 7-amino-5-azaspiro[2.4]heptan-5-yl. The cyclic amino group may have one or more substituents, such as alkyl, hydroxyalkyl, aminoalkyl, acyl, amino, hydroxy, and (5-methyl-2-oxo-1,3-dioxol-4-yl)methyl. The alkyl or acyl moiety in such substituent groups may be those mentioned hereinbefore. Particularly, substituted or unsubstituted pyrrolidinyl and piperazinyl are preferred.

Where the phenyl represented by X is been substituted, the substituent may for example be amino, alkylamino, aminoalkyl, hydroxy, alkoxy, or acyl. The alkyl or alkoxy moiety of such substituent groups may be those mentioned hereinbefore. The acyl may be the same as the acyl moiety of said acyloxy.

The present invention can be carried into practice, for example according to the following reaction schema, to provide optically active compounds [Ia] and [Ib] of the invention. 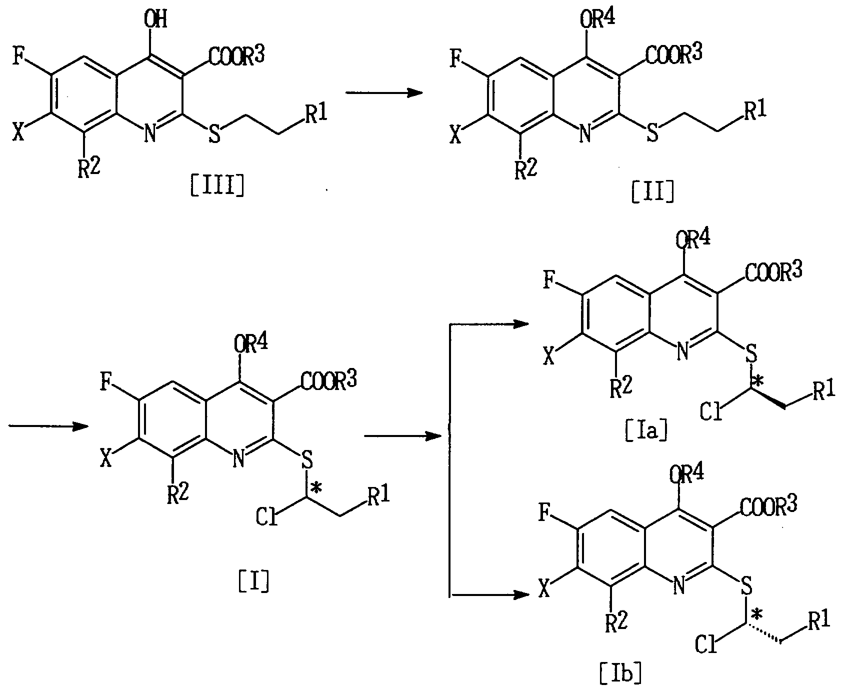 [In the above schema, R¹, R², R³, R⁴, and X are as defined hereinbefore]

Thus, compound [III] is acylated to compound [II] by subjecting the 4-hydroxyl group of compound [III] to react with a chiral carboxylic acid or amino acid, or a reactive derivative thereof (e.g. the corresponding acid chloride or acid anhydride).

When a chiral carboxylic acid or amino acid is used, the acylation of compound [III] is carried out in a suitable solvent in the presence of a suitable condensing agent. The solvent that can be used includes halogenated hydrocarbons such as methylene chloride and chloroform, aromatic hydrocarbons such as benzene and toluene, and hydrocarbons such as n-hexane. As the chiral carboxylic acid or amino acid, the compound corresponding to R⁴ can be employed. For example, 2-phenylpropionic acid, O-methylmandelic acid, O-benzyllactic acid, N-benzyloxycarbonyl-D-phenylglycine, N-benzyloxycarbonyl-L-alanine, N-benzoyl-L-proline, N-benzoyl-L-alanine, N-methoxycarbonyl-D-glycine, O-acetoxy-2-phenylacetic acid, and 2-methyloctylic acid can be employed. The condensing agent that can be used includes acetic anhydride, trifluoroacetic anhydride, dicyclohexylcarbodiimide (DCC), N-ethyl-N'-(3-dimethylaminopropyl)carbodiimide (EDC), trifluoroacetylimidazole, and acetylimidazole, among others. The proportion of the condensing agent and of the carboxylic acid or amino acid per mole of [III] is equimolar to excess, preferably 1.0-2.5 moles. The reaction is usually carried out at a range of -78°C to the boiling point of the solvent, preferably at 0°C - 50°C. The reaction time depends on the species of [III] and of the carboxylic acid or amino acid, the kind of solvent used, and the reaction temperature but is generally 30 minutes to 48 hours.

The acylation of compound [III] with a reactive derivative (e.g. acid chloride or acid anhydride) of said chiral carboxylic acid or amino acid is conducted in a suitable solvent in the presence of a suitable base. As the acid chloride, the acid chloride of said carboxylic acid or amino acid can be employed. As the acid anhydride, the acid anhydride of a chiral carboxylic acid or amino acid, or the mixed acid anhydride available from a chiral carboxylic acid or amino acid and an alkyl chlorocarbonate can be employed. The solvent can be the one mentioned hereinbefore. The base that can be used includes organic bases such as sodium acetate, triethylamine, tripropylamine, pyridine, succinimide sodium and α-pyrrolidone sodium salt, and inorganic bases such as lithium hydroxide, sodium hydrogen carbonate and sodium hydroxide. The proportion of the base and of the acid chloride or acid anhydride per mole of [III] is equimolar to excess, preferably 1.0-2.5 moles. The reaction is generally conducted at a range of -78°C to the boiling point of the solvent, preferably at 0°C - 50°C. The reaction time is dependent on the species of [III], species of acid chloride or acid anhydride of said carboxylic acid or amino acid, the kind of solvent used, and reaction temperature but is generally 30 minutes to 48 hours.

The objective compound [II] thus obtained can be isolated and purified in the form of free base or an acid addition salt by the per se known procedures such as concentration, pH adjustment, redistribution, solvent extraction, crystallization, and chromatography.

The compound [II] is then chlorinated to produce a compound [I] which is a mixture of diastereomers. This chlorination reaction is carried out with a chlorinating agent in a solvent inert to the reaction. The solvent that can be used includes halogenated hydrocarbons such as chloroform, dichloromethane and carbon tetrachloride, hydrocarbons such as hexane, and ethers such as dioxane and tetrahydrofuran. As the chlorinating agent, N-chlorosuccinimide, sulfuryl chloride, or chlorine can be employed. Usually, the chlorinating agent is used in an equimolar proportion to excess, preferably 1.0 - 5.0 equivalents. The reaction is generally conducted at a range of -30°C to 100°C. The reaction time depends on the species of [II], of chlorinating agent, and of solvent used, and reaction time but is generally 30 minutes to 24 hours.

The resulting mixture of two diastereomer esters can be easily resolved into respective diastereomers [Ia] and [Ib] by utilizing the difference in polarity or solubility, for example by chromatography or fractional crystallization.

The fractional crystallization can be carried out by dissolving the mixture of two diastereomer esters in a suitable solvent and subjecting crystals of only one diastereomer esters to deposit selectively under stirring, under cooling or otherwise. The solvent can be selected from a variety of solvents other than alcohols. Thus, for example, ethers such as diethyl ether, diisopropyl ether, dipropyl ether, dioxane and tetrahydrofuran; esters such as ethyl acetate; halogenated hydrocarbons such as chloroform, dichloromethane and carbon tetrachloride; hydrocarbons such as pentane, hexane, cyclohexane, petroleum ether and ligroin, and aprotic polar solvents such as acetonitrile, N,N-dimethylformamide (DMF) and acetone. can be employed. These solvents can be used in admixture. For improvement of purity, the harvested crystals may be recrystallized from the above-mentioned solvent. When the diastereomers are ethyl 6,7-difluoro-2-(1-chloro-2-fluoroethyl)thio-4-((R)-2- phenylpropionyl)oxyquinoline-3-carboxylate and ethyl 6,7-difluoro-2-(1-chloro-2-fluoroethyl)thio-4-((R)-2-methoxy-2-phenylacetoxy)quinoline-3-carboxylate, diisopropyl ether is particularly preferred as the solvent for fractional crystallization.

The starting compound [III] can be produced by the known technology (e.g. WO 93/25532).

The optically active compounds [Ia] and [Ib] of the present invention are new and are of use as intermediates for the synthesis of optically active thiazetoquinolinecarboxylic acid derivatives which are of value for the treatment of infectious diseases caused by gram-negative bacteria, refractory _ Pseudomonas aeruginosa infection, and infectious diseases caused by gram-positive bacteria (WO 94/14819). For example, compound [Ib] of the present invention can be cyclized, by a known process (e.g. WO 93/25532) as shown below, into optically active compound [IVb] which is an important intermediate for the production of thiazetoquinolinecarboxylic acid derivatives having antibacterial activity. 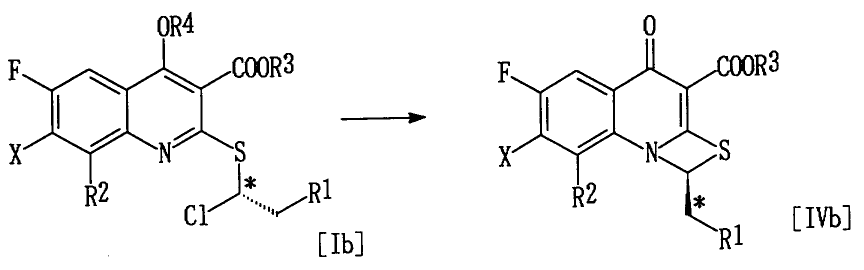 [In the above schema, R¹, R², R³, R⁴, and X are as defined hereinbefore]

Thus, [IVb] can be produced by cyclizing [Ib] in a solvent in the presence of a base generally at room temperature to 100°C. In this process, R⁴ is simultaneously eliminated. The solvent can be any solvent that does not interfere with the reaction. Thus, for example, hydrocarbons such as benzene and toluene, ethers such as tetrahydrofuran and dioxane, aprotic polar solvents such as N,N-dimethylformamide, N,N-dimethylacetamide, dimethyl sulfoxide and sulfolane, or the mixture thereof can be employed. The base may for example be an organic base (e.g. sodium acetate, triethylamine, tripropylamine, pyridine, succinimide sodium salt, α-pyrrolidone sodium salt, etc.) or an inorganic base (e.g. lithium hydroxide, sodium hydrogen carbonate, sodium hydroxide, etc.). The base is generally used in a proportion of 1-10 equivalents relative to [Ib]. The reaction time depends on the species of [Ib], of base, and of solvent, as well as on the reaction temperature but is generally 30 minutes to 180 hours.

### BEST MODE FOR CARRYING OUT THE INVENTION

The production method and compounds [Ia] and [Ib] of the present invention are described in further detail in the following examples which, however, should not be construed as defining the scope of the invention.

### Example 1

### (1) Ethyl 6,7-difluoro-2-(2-fluoroethyl)thio-4-((R)-2-phenylpropionyl)oxyquinoline-3-carboxylate (production of compound [II])

In 45 ml of chloroform was dissolved 2.40 g of ethyl 6,7-difluoro-2-(2-fluoroethyl)thio-4-hydroxyquinoline-3-carboxylate and 1.01 g of triethylamine. To this solution was gradually added a solution of 1.70 g of (R)-(-)-2-phenylpropionyl chloride in 5.0 ml chloroform dropwise under ice-cooling and stirring. Upon completion of dropwise addition, the mixture was stirred at room temperature for 5 hours. After completion of the reaction, the reaction mixture was serially washed with 5% aqueous sodium hydrogen carbonate solution, 1% hydrochloric acid, and saturated aqueous sodium chloride solution, dried over magnesium sulfate, and concentrated. The residue was purified by column chromatography (Wakogel C-200, n-hexane-ethyl acetate = 30:1→2:1) to give 3.12 g of the title compound. Yield 80.0%, m.p. 71-73°C.

| Elemental analysis for C₂₃H₂₀F₃NO₄S | | | |
|---|---|---|---|
| Calcd. (%): | C, 59.60; | H, 4.35; | N, 3.02 |
| Found (%): | C, 59.66; | H, 4.43; | N, 3.04 |

### (2) Ethyl 2-(1-chloro-2-fluoroethyl)thio-6,7-difluoro-4-((R)-2-phenylpropionyl)oxyquinoline-3-carboxylate (the production of compound [I] and of diastereomers by resolution)

In 50 ml of methylene chloride was dissolved 2.35 g of ethyl 6,7-difluoro-2-(2-fluoroethyl)thio-4-((R)-2-phenylpropionyl)oxyquinoline-3-carboxylate obtained in (1) above and 1.37 g of sulfuryl chloride and then the solution was refluxed for 2 hours. After completion of the reaction, the reaction mixture was concentrated under reduced pressure and the residue was purified by column chromatography (Wakogel C-200, n-hexane-ethyl acetate = 30:1→20:1) to give 2.10 g of crude crystals (yield 83.3%). The progress of reaction was monitored by HPLC.

### Conditions of HPLC

Column: YMC AM-302 (ODS) Φ 4.6 x 250 mm
Mobile phase: Acetonitrile-water-methanesulfonic acid = 70:30:0.5
Flow rate: 1.0 ml/min.
Detector: Shimadzu UV detector SPD-6A, wavelength 254 nm

The above crystal crop was recrystallized from about 30 ml of isopropyl ether for diastereomer resolution to provide 840 mg of crystals of ethyl 2-(1-chloro-2-fluoroethyl)thio-6,7-difluoro-4-((R)-2-phenylpropionyl)oxyquinoline-3-carboxylate. The compound thus obtained by resolution was quantitated using an HPLC column.

### Conditions of HPLC

Column: Daicel Chiral Cell OD Φ 4.6 x 250 mm
Mobile phase: Hexane-2-propanol = 70:30
Flow rate: 0.8 ml/min.
Detector: Shimadzu UV detector SPD-6A, wavelength 254 nm
Yield: 40.0%, diastereomer excess (d.e.): 52.9%

This crystal crop was recrystallized from about 15 ml of isopropyl ether again for diastereomer resolution to provide 520 mg of crystals of the title compound.
Yield: 61.9%, d.e. 79.1%

The above crystal crop was further recrystallized from about 15 ml of isopropyl ether for diastereomer resolution to provide 310 mg of crystals.
Yield: 59.6%, d.e.: 96.3%
m.p. 108-109°C

| Elemental analysis for C₂₃H₁₉ClF₃NO₄S | | | |
|---|---|---|---|
| Calcd. (%): | C, 55.48; | H, 3.85; | N, 2.81 |
| Found (%): | C, 55.72; | H, 3.52; | N, 2.74 |

### Example 2

### (1) Ethyl 4-[(N-benzyloxycarbonyl)-D-phenylglycyl]oxy-6,7-difluoro-2-(2-fluoroethyl)thioquinoline-3-carboxylate

In 15 ml of methylene chloride was dissolved 1.66 g of ethyl 6,7-difluoro-2-(2-fluoroethyl)thio-4-hydroxyquinoline-3-carboxylate and 2.85 g of N-benzyloxycarbonyl-D-α-phenylglycine. To this solution was added a solution of 2.06 g DCC in 5 ml methylene chloride dropwise under ice-cooling and stirring and the mixture was further stirred at room temperature for 20 hours. The N,N'-dicyclohexylurea (DCU) separating out from the solution was filtered off and the filtrate was concentrated and recrystallized from ethyl acetate to provide 2.0 g of colorless powders.
Yield 66.8% m.p. 148°C

| Elemental analysis for C₃₀H₂₅F₃N₂O₆S | | | |
|---|---|---|---|
| Calcd. (%): | C, 60.20; | H, 4.21; | N, 4.67 |
| Found (%): | C, 60.77; | H, 4.60; | N, 5.06 |

### (2) Ethyl 4-[(N-benzyloxycarbonyl)-D-phenylglycyl]oxy-2-(1-chloro-2-fluoroethyl)thio-6,7-difluoroquinoline-3-carboxylate

In 20 ml of methylene chloride was dissolved 2.0 g of ethyl 4-[(N-benzyloxycarbonyl)-D-phenylglycyl]oxy-6,7-difluoro-2-(2-fluoroethyl)thioquinoline-3-carboxylate obtained in (1) above. To this solution was added 0.90 g of sulfuryl chloride dropwise with ice-cooling and stirring and the mixture was reacted under the same conditions for 1 hour and then at room temperature for 17 hours. This reaction mixture was concentrated under reduced pressure and the residue was purified by column chromatography (Wakogel C-200, n-hexane-ethyl acetate = 100:1→50:1) to provide 1.0 g of the title compound. m.p. 176°C. The compound obtained by resolution was quantitated using an HPLC column.

### Conditions of HPLC

Column: YMC AM-302 (ODS)Φ 4.6 x 250 mm
Mobile phase: Acetonitrile-water-methanesulfonic acid = 70:30:0.5
Flow rate: 1.0 ml/min.
Detector: Shimadzu UV detector SPD-6A, wavelength 254 nm
d.e.: 32.4%

| Elemental analysis for C₃₀H₂₄ClF₃N₂O₆S | | | |
|---|---|---|---|
| Calcd. (%): | C, 56.92; | H, 3.82; | N, 4.43 |
| Found (%): | C, 56.45; | H, 3.88; | N, 4.41 |

### Example 3

### (1) Ethyl 4-[(N-benzyloxycarbonyl)-L-alanyl]oxy-6,7-difluoro-2-(2-fluoroethyl)thioquinoline-3-carbonylate

A suspension of 1.00 g ethyl 6,7-difluoro-2-(2-fluoroethyl)thio-4-hydroxyquinoline-3-carboxylate and 0.67 g N-benzyloxycarbonyl-L-alanine in ethyl acetate (30 ml) was prepared under ice-cooling and stirring. To this suspension was added 0.62 g of DCC and the mixture was stirred at room temperature for 20 hours. DCU precipitated out from the solution was filtered off and the filtrate was washed well with 1% hydrochloric acid, 1% aqueous sodium hydrogen carbonate solution, and saturated aqueous sodium chloride solution in that order, dried over magnesium sulfate, and concentrated. The residue was purified by column chromatography (Wakogel C-200, chloroform and chloroform-methanol = 100:1) to provide 1.14 g of the title compound.
Yield 72.6% m.p. 134-135°C

| Elemental analysis for C₂₅H₂₃F₃N₂O₆S | | | |
|---|---|---|---|
| Calcd. (%): | C, 55.97; | H, 4.32; | N, 5.22 |
| Found (%): | C, 56.29; | H, 4.48; | N, 5.29 |

### (2) Ethyl 4-[(N-benzyloxycarbonyl)-L-alanyl]oxy-2-(1-chloro-2-fluoroethyl)thio-6,7-difluoroquinoline-3-carboxylate

In 20 ml of methylene chloride was dissolved 960 mg of ethyl 4-[(N-benzyloxycarbonyl)-L-alanyl]oxy-6,7-difluoro-2-(2-fluoroethyl)thioquinoline-3-carboxylate obtained in (1) above and 500 mg of sulfuryl chloride and the mixture was refluxed for 2 hours. After completion of the reaction, the reaction mixture was concentrated under reduced pressure and the residue was purified by column chromatography (Wakogel C-200, chloroform) to provide 640 mg of crude crystals.
Yield 62.7%

This crystal crop was recrystallized from 30 ml of isopropyl ether-ethyl acetate (1:1) for diastereomer resolution to provide 420 mg of crystals. Yield 65.6%

The compound obtained by resolution was quantitated by HPLC under the same conditions as described in Example 2 (2). d.e.: 38.5%

This crystal crop was further recrystallized from 30 ml of isopropyl ether-ethyl acetate (1:1) for diastereomer resolution to give 260 mg of crystals.
Yield 61.9%

The quantitation was carried out under the same HPLC conditions as above. d.e.: 50.1%

The above crystal crop was further recrystallized from 20 ml of isopropyl ether-ethyl acetate (1:1) for diastereomer resolution to provide 140 mg of crystals. Yield 53.8%

The quantitation was carried out under the same HPLC conditions as in Example 2 (2). d.e.: 58.4%
m.p. 145-146°C

| Elemental analysis for C₂₅H₂₂ClF₃N₂O₆S | | | |
|---|---|---|---|
| Calcd. (%): | C, 52.59; | H, 3.88; | N, 4.90 |
| Found (%): | C, 52.44; | H, 3.90; | N, 5.08 |

### Example 4

### (1) Methyl 6,7-difluoro-2-(2-fluoroethyl)thio-4-((R)-2-phenylpropionyl)oxyquinoline-3-carboxylate

Using 1.50 g of methyl 6,7-difluoro-2-(2-fluoroethyl)thio-4-hydroxyquinoline-3-carboxylate and 0.96 g of (R)-(-)-phenylpropionyl chloride, the procedure of Example 1 (1) was carried out in the same manner to provide 1.46 g of the title compound as colorless crystals.
m.p. 247-249°C
Optical rotation [α]_{D}=-49.56° (c=1.138, CHCl₃)

| Elemental analysis for C₂₂H₁₈F₃NO₄S | | | |
|---|---|---|---|
| Calcd. (%): | C, 58.79; | H, 4.04; | N, 3.12 |
| Found (%): | C, 58.70; | H, 4.10; | N, 3.26 |

### (2) Methyl 2-(1-chloro-2-fluoroethyl)thio-6,7-difluoro-4-((R)-2-phenylpropionyl)oxyquinoline-3-carboxylate

Using the compound obtained in (1) above, the procedure of Example 1 (2) was carried out in the same manner to provide 0.97 g of the title compound as colorless crystals. This crystal crop was recrystallized from isopropyl ether twice for diastereomer resolution. The diastereomer ratio was analyzed by HPLC.
Yield: 20.6%, d.e.: 20.6%

### Conditions of HPLC analysis

Apparatus: LC-10A (Shimadzu)
Column: YMC-Pac ODS-AM (AM-302) 150x4.6 mmI.D.
Detector: UV (254 nm)
Flow rate: 1.0 ml/min.
Eluent: MeCN-H₂O-MeSO₃H = 70:30:0.1
m.p. 84-85°C
Optical rotation [α]_{D}=-60.25° (c=1.029, CHCl₃)

| Elemental analysis for C₂₂H₁₇ClF₃NO₄S | | | |
|---|---|---|---|
| Calcd. (%): | C, 54.67, | H, 3.54; | N, 2.89 |
| Found (%): | C, 54.76; | H, 3.51; | N, 2.95 |

### Example 5

### (1) n-Propyl 6,7-difluoro-2-(2-fluoroethyl)thio-4-((R)-2-phenylpropionyl)oxyquinoline-3-carboxylate

Using 1.50 g of n-propyl 6,7-difluoro-2-(2-fluoroethyl)thio-4-hydroxyquinoline-3-carboxylate and 0.88 g of (R)-(-)-phenylpropionyl chloride, the procedure of Example 1 (1) was carried out in the same manner to provide 2.01 g of the title compound.
¹H-NMR (CDCl₃) δppm: 1.03 (3H, t, J=7.0), 1.69 (3H, d, J=6.9), 1.85 (2H, tq, J=7.0, 6.5), 3.61 (2H, dt, J=19.3, 6.4), 4.11 (1H, q, J=6.9), 4.36 (2H, t, J=6.5), 4.63 (2H, dt, J=46.9, 6.4), 6.73 (1H, dd, J=10.6, 8.2), 7.43-7.47 (5H, m), 7.65 (1H, dd, J= 11.1, 7.6).

### (2) n-Propyl 2-(1-chloro-2-fluoroethyl)thio-6,7-difluoro-4-((R)-2-phenylpropionyl)oxyquinoline-3-carboxylate

Using 1.70 g of the compound obtained in (1) above, the procedure of Example 1 (2) was carried out in the same manner to provide 1.34 g of the title compound. This crystal crop was recrystallized from isopropyl ether 3 times for diastereomer resolution. The diastereomer ratio was analyzed under the same HPLC conditions as in Example 4 (2) except that MeCN-H₂O-MeSO₃H = 80:20:0.1 was used as the eluent.
Yield: 34.6% d.e.: 46%
m.p. 94-95°C
Optical rotation [α]_{D}=53.70° (c=1.054, CHCl₃)

| Elemental analysis for C₂₄H₂₁ClF₃NO₄S | | | |
|---|---|---|---|
| Calcd. (%): | C, 56.31; | H, 4.13; | N, 2.74 |
| Found (%): | C, 56.29; | H, 4.06; | N, 2.90 |

### Example 6

### (1) Isopropyl 6,7-difluoro-2-(2-fluoroethyl)thio-4-((R)-2-phenylpropionyl)oxyquinoline-3-carboxylate

Using 2.00 g of isopropyl 6,7-difluoro-2-(2-fluoroethyl)thio-4-hydroxyquinoline-3-carboxylate and 1.17 g of (R)-(-)-2-phenylpropionyl chloride, the procedure of Example 1 (1) was carried out in the same manner to provide 2.57 g of the title compound.
m.p. 56-58°C
Optical rotation [α]_{D}=-62.12° (c=1.027, CHCl₃)

| Elemental analysis for C₂₄H₂₂F₃NO₄S | | | |
|---|---|---|---|
| Calcd. (%): | C, 60.37; | H, 4.64; | N, 2.93 |
| Found (%): | C, 60.21; | H, 4.52; | N, 3.08 |

### (2) Isopropyl 2-(1-chloro-2-fluoroethyl)thio-6,7-difluoro-4-((R)-2-phenylpropionyl)oxyquinoline-3-carboxylate

Using 2.50 g of the compound obtained in (1) above, the procedure of Example 1 (2) was carried out in the same manner to provide 2.60 g of the title compound. This crystal crop was recrystallized from n-hexane 4 times for diastereomer resolution. The diastereomer ratio was analyzed by HPLC under the same conditions as used in Example 5 (2).
Yield: 52.2% d.e.: 30.8%
m.p. 91-92°C
Optical rotation [α]_{D}=29.24° (c=1.053, CHCl₃)

| Elemental analysis for C₂₄H₂₁ClF₃NO₄S | | | |
|---|---|---|---|
| Calcd. (%): | C, 56.31; | H, 4.13; | N, 2.74 |
| Found (%): | C, 56.45; | H, 4.00; | N, 2.91 |

### Example 7

### (1) Ethyl 4-[(O-benzyl)-L-lactoyl]oxy-6,7-difluoro-2-(2-fluoroethyl)thioquinoline-3-carboxylate

Using 2.43 g of ethyl 6,7-difluoro-2-(2-fluoroethyl)thio-4-hydroxyquinoline-3-carboxylate and 1.75 g of O-benzyl-L-lactoyl chloride, the procedure of Example 1 (1) was carried out in the same manner to provide 2.87 g of the title compound. m.p. 45-47°C
Optical rotation [α]_{D}=-47.35° (c=1.136, CHCl₃)

| Elemental analysis for C₂₄H₂₂F₃NO₅S | | | |
|---|---|---|---|
| Calcd. (%): | C, 58.41; | H, 4.49; | N, 2.84 |
| Found (%): | C, 58.40; | H, 4.39; | N, 2.88 |

### (2) Ethyl 4-[(O-benzyl)-L-lactoyl]oxy-2-(1-chloro-2-fluoroethyl)thio-6,7-difluoroquinoline-3-carboxylate

Using 2.00 g of the compound obtained in (1) above, the procedure of Example 1 (2) was carried out in the same manner to provide 1.87 g of the title compound. This crystal crop was recrystallized from isopropyl ether twice for diastereomer resolution.
Yield: 99.5%. The d.e. could not be determined because of overlap of peaks.
m.p. 83-84°C
Optical rotation [α]_{D}=-63.87° (c=1.099, CHCl₃)

| Elemental analysis for C₂₄H₂₁ClF₃NO₅S | | | |
|---|---|---|---|
| Calcd. (%): | C, 54.60; | H, 4.01; | N, 2.65 |
| Found (%): | C, 54.57; | H, 3.88; | N, 2.74 |

### Example 8

### (1) Ethyl 4-[(2-acetoxy-2-phenyl)acetoxy-6,7-difluoro-2-(2-fluoroethyl)thioquinoline-3-carboxylate

Using 4.97 g of ethyl 6,7-difluoro-2-(2-fluoro ethyl)thio-4-hydroxyquinoline-3-carboxylate and 3.83 g of S-(+)-2-acetoxy-2-phenylacetyl chloride, the procedure of Example 1 (1) was carried out in the same manner to provide 2.34 g of the title compound. m.p. 83°C
¹H-NMR (CDCl₃) δppm: 1.25 (3H, t, J=7.0), 2.27 (3H, s), 3.59 (2H, dt, J=18.2, 7.0), 4.11 (2H, q, J=7.0), 4.67 (2H, dt, J=46.9, 7.0), 6.26 (1H, s), 7.10 (1H, dd, J=10.6, 8.2), 7.44-7.60 (5H, m), 7.72 (1H, dd, J=10.6, 7.0).

### (2) Ethyl (4S)-[(2-acetoxy-2-phenyl)acetoxy-2-(1-chloro-2-fluoroethyl)thio-6,7-difluoroquinoline-3-carboxylate

Using 2.34 g of the compound obtained in (1) above, the procedure of Example 1 (2) was carried out in the same manner to provide 1.57 g of the title compound. This crystal crop was recrystallized from isopropyl ether 3 times for diastereomer resolution.
Yield: 64.3%. The d.e. could not be determined due to an overlap of peaks.
m.p. 109°C
¹H-NMR (CDCl₃) δppm: 1.24 (3H, t, J=7.0), 2.26 (3H, s), 4.12 (2H, m), 4.90 (2H, m), 6.29 (1H, s), 6.60 (1H, m), 7.10 (1H, dd, J=10.0, 8.2), 7.67 (1H, dd, J=10.8, 7.2)

### Example 9

### (1) Ethyl 6,7-difluoro-2-(2-fluoroethyl)thio-4-((R)2-methoxy-2-phenyl)acetoxyquinoline-3-carboxylate

Using 2.49 g of ethyl 6,7-difluoro-2-(2-fluoroethyl)thio-4-hydroxyquinoline-3-carboxylate and 1.50 g of (R)-(-)-2-methoxy-2-phenylacetyl chloride, the procedure of Example 1 (1) was carried out in the same manner to provide 2.80 g of the title compound. m.p. 88-90°C
Optical rotation [α]_{D}=67.61° (c=0.562, CHCl₃)

| Elemental analysis for C₂₃H₂₀F₃NO₅S | | | |
|---|---|---|---|
| Calcd. (%): | C, 57.62; | H, 4.20; | N, 2.92 |
| Found (%): | C, 57.67; | H, 4.08; | N, 3.07 |

### (2) Ethyl 2-(1-chloro-2-fluoroethyl)thio-6,7-difluoro-4-((R)-2-methoxy-2-phenyl)acetoxyquinoline-3-carboxylate

Using 2.50 g of the compound obtained in (1) above, the procedure of Example 1(2) was carried out in the same manner to provide 1.55 g of the title compound. This crystal crop was recrystallized from isopropyl ether 3 times for diastereomer resolution. The diastereomer ratio was analyzed by HPLC under the same conditions as in Example 4 (2).
Yield: 40.0%, d.e.: >99.9%.
m.p. 105-106°C
Optical rotation [α]_{D}=64.29° (c=1.294, CHCl₃)

| Elemental analysis for C₂₃H₁₉ClF₃NO₅S | | | |
|---|---|---|---|
| Calcd. (%): | C, 53.75; | H, 3.73; | N, 2.73 |
| Found (%): | C, 53.99; | H, 3.71; | N, 3.01 |

### Example 10

### (1) Ethyl 4-[(N-benzyloxycarbonyl)-L-alanyl]oxy-6,7-difluoro-2-(2-fluoroethyl)thioquinoline-3-carboxylate

A 50 ml eggplant-shaped flask was charged with 1.00 g of ethyl 6,7-difluoro-2-(2-fluoroethyl)thio-4-hydroxyquinoline-3-carboxylate, 0.67 g of benzyloxycarbonyl-L-alanine, and 30 ml of ethyl acetate, followed by addition of 0.62 g of DCC with ice-cooling and vigorous stirring. The reaction system was brought back to room temperature and the reaction was further carried out for 20 hours. This reaction mixture was filtered and the filtrate was washed serially with 1% aqueous HCl solution, 1% aqueous NaHCO₃ solution, and saturated NaCl solution, dried over MgSO₄, and concentrated. The residue was purified by silica gel column chromatography (Wakogel C-200, CHCl₃/MeOH = 100:1) to provide 1.14 g of the title compound as colorless crystals. m.p. 134-135°C
Optical rotation [α]_{D}=-10.69° (c=0.804, CHCl₃)

| Elemental analysis for C₂₅H₂₃F₃N₂O₆S | | | |
|---|---|---|---|
| Calcd. (%): | C, 55.97; | H, 4.32; | N, 5.22 |
| Found (%): | C, 56.29; | H, 4.48; | N, 5.24 |

### (2) Ethyl 4-[(N-benzyloxycarbonyl)-L-alanyl]oxy-2-(1-chloro-2-fluoroethyl)thio-6,7-difluoroquinoline-3-carboxylate

Using 0.96 g of the compound obtained in (1) above, the procedure of Example 1 (2) was carried out in the same manner to provide 0.64 g of the title compound. This crystal crop was recrystallized from isopropyl ether-ethyl acetate 3 times for diastereomer resolution. The diastereomer ratio was analyzed by HPLC under the same conditions as in Example 4 (2) using MeCN:H₂O:MeSO₃H = 75:25:0.1 as the eluent.
Yield: 43.8%, d.e.: 58.4%
m.p. 145-146°C
Optical rotation [α]_{D}=63.12° (c=1.017, CHCl₃)

| Elemental analysis for C₂₅H₂₂ClF₃N₂O₆S | | | |
|---|---|---|---|
| Calcd. (%): | C, 52.59; | H, 3.88; | N, 4.90 |
| Found (%): | C, 52.44; | H, 3.90; | N, 5.08 |

### Example 11

### (1) Ethyl 4-[(N-benzyloxycarbonyl)-D-phenylglycyl]oxy-6,7-difluoro-2-(2-fluoroethyl)thioquinoline-3-carboxylate

Using 1.66 g of ethyl 6,7-difluoro-2-(2-fluoroethyl)thio-4-hydroxyquinoline-3-carboxylate and 2.85 g of benzyloxycarbonyl-D-phenylglycine, the procedure of Example 10 (1) was carried out in the same manner to provide 2.00 g of the title compound. m.p. 148°C
Optical rotation [α]_{D}=-65.55° (c=1.083, CHCl₃)

| Elemental analysis for C₃₀H₂₅F₃N₂O₆S | | | |
|---|---|---|---|
| Calcd. (%): | C, 60.20; | H, 4.21; | N, 4.67 |
| Found (%): | C, 60.77; | H, 4.60; | N, 5.24 |

### (2) Ethyl 4-[(N-benzyloxycarbonyl)-D-phenylglycyl]oxy-2-(1-chloro-2-fluoroethyl)thio-6,7-difluoroquinoline-3-carboxylate

Using 2.00 g of the compound obtained in (1) above, the procedure of Example 1 (2) was carried out in the same manner to provide 1.00 g of the title compound. This crystal crop was recrystallized from acetone 4 times for diastereomer resolution. The diastereomer ratio was analyzed by HPLC under the same conditions as in Example 4 (2).
Yield: 45.0%, d.e.: 32.4%
m.p. 176°C
Optical rotation [α]_{D}=-17.38° (c=1.070, CHCl₃)

| Elemental analysis for C₃₀H₂₄ClF₃N₂O₆S | | | |
|---|---|---|---|
| Calcd. (%): | C, 56.92; | H, 3.82; | N, 4.43 |
| Found (%): | C, 56.45; | H, 3.88; | N, 4.41 |

### Reference Example 1

### Ethyl (-)-6,7-difluoro-1-fluoromethyl-4-oxo-4H-[1,3]thiazeto[3,2-a]quinoline-3-carboxylate

A solution prepared by mixing 150 mg of the 2-(1-chloro-2-fluoroethyl)thio-6,7-difluoro-4-((R)-2- phenylpropionyl)oxyquinoline-3-carboxylate obtained in Example 1 (2) with 90 mg of triethylamine, 6 mg of water, and 3.0 ml of tetrahydrofuran (THF) was reacted under reflux for 6 hours. After completion of the reaction, the reaction mixture was gradually cooled to room temperature and stirred at room temperature for 1 hour and the resulting crystals were collected by filtration. The crystal crop was thoroughly washed with THF, water, THF, and ether in the order mentioned and dried in vacuo to provide 83 mg of the title compound.
Yield 83.8%
m.p. 209-210°C
Optical rotation [α]_{D}=-103.68° (DMF, c=0.814, at 20°C)

| Elemental analysis for C₁₄H₁₀F₃NO₃S | | | |
|---|---|---|---|
| Calcd. (%): | C, 51.06; | H, 3.06; | N, 4.25 |
| Found (%): | C, 50.86; | H, 3.15; | N, 4.39 |

The compound obtained by the above reaction was quantitated by HPLC using a chiral column.

### Conditions of HPLC

Column: Daicel Chiral Cell ODΦ I 4.6x250 mm
Mobile phase: n-Hexane-isopropyl alcohol = 70:30
Flow rate: 0.8 ml/min.
Detector: Shimadzu UV detector SPD-6A, wavelength 254 nm
Optical purity: 91.0%

### Reference Example 2

### Ethyl (-)-6,7-difluoro-1-fluoromethyl-4-oxo-4H-[1,3]thiazeto[3,2-a]quinoline-3-carboxylate

Using the ethyl 2-(1-chloro-2-fluoroethyl)thio-6,7-difluoro-4-((R)2-phenylpropionyl)oxyquinoline-3-carboxylate obtained in Example 2 (2), the procedure of Reference Example 1 was carried out in the same manner to provide the title compound.

### Reference Example 3

### Ethyl (-)-6,7-difluoro-1-fluoromethyl-4-oxo-4H-[1,3]thiazeto[3,2-a]quinoline-3-carboxylate

Using the ethyl 4-[(R)-(N-benzyloxycarbonyl)-L-alanyl]oxy-2-(1-chloro-2-fluoroethyl)thio-6,7-difluoroquinoline-3-carboxylate obtained in Example 3 (2), the procedure of Reference Example 1 was carried out in the same manner to provide the title compound.

### Reference Example 4

### Ethyl (-)-6,7-difluoro-1-fluoromethyl-4-oxo-4H-[1,3]thiazeto[3,2-a]quinoline-3-carboxylate

Using the ethyl 2-(1-chloro-2-fluoroethyl)thio-6,7-difluoro-4-((R)2-methoxy-2-phenyl)acetoxyquinoline- 3-carboxylate obtained in Example 9 (2), the procedure of Reference Example 1 was carried out in the same manner to provide the title compound. Optical purity 97.1%

### INDUSTRIAL APPLICABILITY

In accordance with the production technology of the present invention, optically active 2-(1-chloro(-2-substituted)ethyl)thio-6-fluoro-7-substituted-4-acyloxyquinoline-3-carboxylic acid alkyl ester derivatives [Ia] and [Ib] can be produced with high efficiency on a mass scale. Moreover, the use of the optically active compound [Ia] or [Ib] of the invention as an intermediate is industrially advantageous in that optically active 6-fluoro-7-halo-1-(substituted)methyl-4-oxo-4H-[1,3]thiazeto[3,2-a]quinolinecarboxylic acid alkyl esters can be produced with high efficiency and economically.

## Claims

1. A process for producing an optically active quinolinecarboxylic acid derivative characterized by reacting a compound of the following general formula [III] 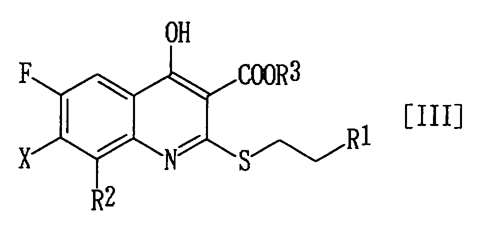 with a chiral carboxylic acid or amino acid, or a reactive derivative thereof, reacting the resulting compound of general formula [II] 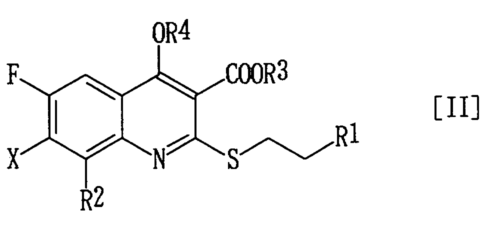 with a chlorinating agent to give a diastereomer mixture [I], 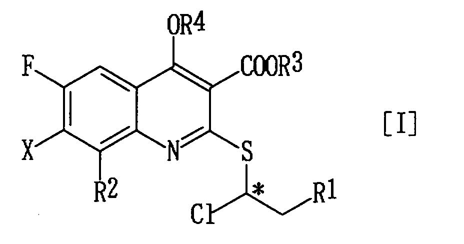 and separating each diastereomer from [I] to provide a compound of general formula [Ia] or [Ib] 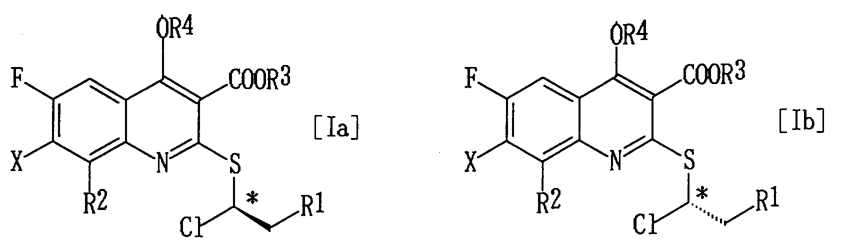 wherein
R¹ represents hydrogen, halogen, hydroxy, alkoxy, aryloxy, or acyloxy;
R² represents hydrogen, alkyl, alkoxy, amino, nitro, or halogen;
R³ represents alkyl;
R⁴ represents chiral acyl;
X represents halogen, substituted or unsubstituted cyclic amino, or phenyl.

2. A process for producing an optically active quinolinecarboxylic acid derivative characterized by reacting a compound of general formula [II] 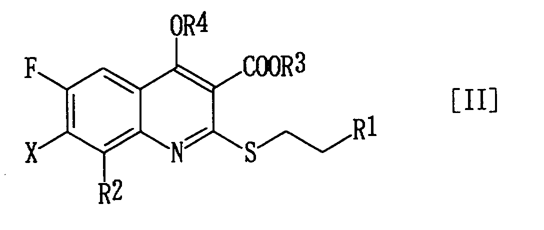 with a chlorinating agent to give a diastereomer mixture [I] 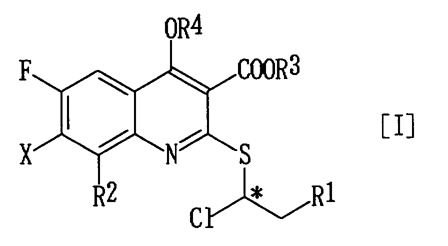 and separating each diastereomer to provide a compound of general formula [Ia] or [Ib] 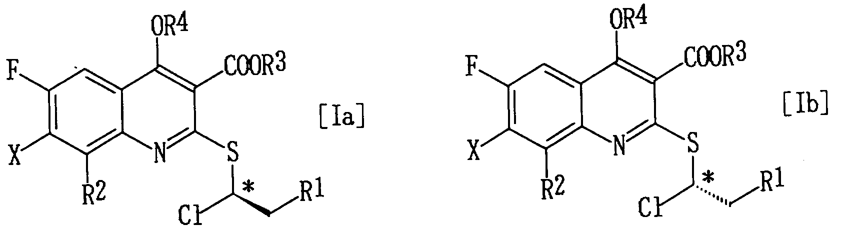 wherein
R¹ represents hydrogen, halogen, hydroxy, alkoxy, aryloxy, or acyloxy;
R² represents hydrogen, alkyl, alkoxy, amino, nitro, or halogen;
R³ represents alkyl;
R⁴ represents chiral acyl;
X represents halogen, substituted or unsubstituted cyclic amino, or phenyl.

3. The process according to Claim 1 or 2 wherein R⁴ represents 2-phenylpropionyl or 2-methoxy-2-phenylacetyl.

4. An optically active quinolinecarboxylic acid derivative of the following general formula [Ia] 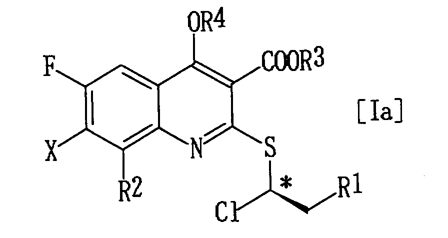 wherein
R¹ represents hydrogen, halogen, hydroxy, alkoxy, aryloxy, or acyloxy;
R² represents hydrogen, alkyl, alkoxy, amino, nitro, or halogen;
R³ represents alkyl;
R⁴ represents chiral acyl;
X represents halogen, substituted or unsubstituted cyclic amino, or phenyl.

5. An optically active quinolinecarboxylic acid derivative of the following general formula [Ib]. 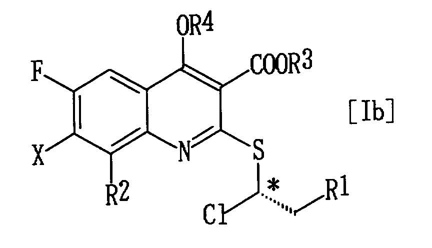 wherein
R¹ represents hydrogen, halogen, hydroxy, alkoxy, aryloxy, or acyloxy;
R² represents hydrogen, alkyl, alkoxy, amino, nitro, or halogen;
R³ represents alkyl;
R⁴ represents chiral acyl;
X represents halogen, substituted or unsubstituted cyclic amino, or phenyl.

6. The quinolinecarboxylic acid derivative of Claim 4 or 5 wherein R⁴ represents 2-phenylpropionyl or 2-methoxy-2-phenylacetyl.
